# EUROPEAN PATENT SPECIFICATION

(11) **EP 3 283 089 B1**
(45) Date of publication and mention of the grant of the patent: **16.03.2022**
(21) Application number: 16779711.7
(22) Date of filing: 13.04.2016
(51) Int. Cl.: A61K 36/02, C12N 1/12, A61K 31/336

(54) **COMPOSITIONS COMPRISING CAROTENOIDS AND USE THEREOF**
ZUSAMMENSETZUNGEN MIT CAROTINOIDEN UND VERWENDUNGEN DAVON
COMPOSITIONS COMPRENANT DES CAROTÉNOÏDES ET UTILISATION DE CELLES-CI

(30) Priority: 13.04.2015 US 201562146469 P; 08.02.2016 US 201662292421 P
(43) Date of publication of application: 21.02.2018
(73) Proprietor: Algatechnologies Ltd., 8884000 Kibbutz Ketura (IL)
(72) Inventor: GRUNDMAN, Omer, 8499000 Midreshet Ben-Gurion (IL); RICHTER, Hadas, 7680300 Beit-Elazari (IL); INI, Santiago, 3440450 Haifa (IL)
(74) Representative: Boult Wade Tennant LLP
(86) International application number: PCT/IL2016/050389
(87) International publication number: WO 2016/166755

(56) References cited:
- EP-A1- 3 116 518
- WO-A1-2006/077433
- WO-A1-2012/047120
- US-A1- 2011 111 038
- US-A1- 2015 044 737
- SANG MIN KIM ET AL: "Fucoxanthin as a major carotenoid in Isochrysis aff. galbana: Characterization of extraction for commercial application", JOURNAL OF THE KOREAN SOCIETY FOR APPLIED BIOLOGICAL CHEMISTRY, vol. 55, no. 4, 1 August 2012 (2012-08-01) , pages 477-483, XP055049996, ISSN: 1738-2203, DOI: 10.1007/s13765-012-2108-3
- SONG XIA ET AL: "Production, Characterization, and Antioxidant Activity of Fucoxanthin from the Marine Diatom Odontella aurita", MARINE DRUGS, vol. 11, no. 7, 23 July 2013 (2013-07-23), pages 2667-2681, XP055519743, DOI: 10.3390/md11072667
- SANG MIN KIM ET AL: "A Potential Commercial Source of Fucoxanthin Extracted from the Microalga", APPLIED BIOCHEMISTRY AND BIOTECHNOLOGY ; PART A: ENZYME ENGINEERING AND BIOTECHNOLOGY, HUMANA PRESS INC, NEW YORK, vol. 166, no. 7, 28 February 2012 (2012-02-28), pages 1843-1855, XP035042483, ISSN: 1559-0291, DOI: 10.1007/S12010-012-9602-2
- IZABELA MICHALAK ET AL: "Algal extracts: Technology and advances", ENGINEERING IN LIFE SCIENCES, vol. 14, no. 6, 18 September 2014 (2014-09-18), pages 581-591, XP055365467, DE ISSN: 1618-0240, DOI: 10.1002/elsc.201400139
- MICHAEL A. BOROWITZKA: "High-value products from microalgae-their development and commercialisation", JOURNAL OF APPLIED PHYCOLOGY, vol. 25, no. 3, 29 January 2013 (2013-01-29), pages 743-756, XP055203653, ISSN: 0921-8971, DOI: 10.1007/s10811-013-9983-9
- ZHAO, PEIPEI ET AL.: 'Silicon enhances the growth of Phaeodactylum tricornutum Bohlin under green light and low temperature.' SCIENTIFIC REPORTS4, [Online] vol. 4, no. 3958, 04 February 2014, pages 1 - 10, XP055323768 Retrieved from the Internet: <URL:http://www. nature .com/articles/srep03958?WT. ec_id=SREP-631-20140211> [retrieved on 2016-07-18]
- BENAVIDES, ANA M. SILVA ET AL.: 'Productivity and biochemical composition of Phaeodactylum tricornutum (Bacillariophyceae) cultures grown outdoors in tubular photobioreactors and open ponds.' BIOMASS AND BIOENERGY, [Online] vol. 54, 18 April 2013, pages 115 - 122, XP028567981 Retrieved from the Internet: <URL:https://www.researchgate.net/profile/G iuseppe_Torzillo/ publication/257421321_Productivity_and_ biochemical _composition_of_Phaeodactylum_trico nutum_(Bacillariophyceae) _cultures_grown_outdoors_in_tubular_photobi oreactors_and_open_ponds/ links/550810f30cf26ff55f7fcd3c.pdf> [retrieved on 2016-07-18]
- Hideki Kanda ET AL: "Extraction of Fucoxanthin from Raw Macroalgae excluding Drying and Cell Wall Disruption by Liquefied Dimethyl Ether", Marine Drugs, vol. 12, no. 5, 30 April 2014 (2014-04-30) , pages 2383-2396, XP055589074, Basel, CH ISSN: 1660-3397, DOI: 10.3390/md12052383

## Description

### FIELD OF INVENTION

The present invention is directed to microalgae extract and microalgae dried biomass compositions including but not limited to, extracts comprising carotenoids and/or fatty acids.

### BACKGROUND OF THE INVENTION

Microalgae grow in either marine or freshwater systems. They are unicellular species which exist individually, or in chains or groups. Microalgae are capable of performing photosynthesis, and are primary producers in the oceans that convert water and carbon dioxide to biomass and oxygen. Microalgae species produce unique products such as carotenoids, antioxidants, fatty acids, enzymes, polymers, peptide, toxins and sterols.

Diatoms are microalgae, composed of a cell wall made primarily of silica and are mainly photosynthetic. Major pigments of diatoms are chlorophylls a and c, beta-carotene, fucoxanthin, diatoxanthin and diadinoxanthin.

Therapeutic supplements from micro-algae comprise an important market in which compounds such as β-carotene, astaxanthin, polyunsaturated fatty acid (PUFA) such as docosahexaenoic acid (DHA) and eicosapentaenic acid (EPA,) and polysaccharides such as β-glucan dominate.

Fucoxanthin is a carotenoid that exhibits anticancer, antioxidant, anti-diabetic, anti-obesity and anti-inflammatory properties. There is a need for microalgae biomass comprising high levels of fucoxanthin, alone or combined with additional carotenoids and/or fatty acids of nutraceutical value.

Further, extraction of fucoxanthin possess a challenge since it is unstable with respect to pH, temperature and light. In addition, when extracted from macro-algae the resulting extract contain heavy metals and iodine, obtained by the macro-algae. Therefore, there is a need for improved extracts comprising substantial amount of fucoxanthin which are essentially free of heavy metals and iodine and have low levels of saccharides.

Sang Min Kim et al. (2012) "Fucoxanthin as a major carotenoid in Isochrysis aff. galbana: Characterization of extraction for commercial application", Journal of the Korean Society for Applied Biological Chemistry, vol. 55, no. 4, pages 477-483, describes a composition comprising a microalga extract comprising fucoxanthin, obtained from *Phaeodactylum tricornutum.*

Song Xia et al. (2013) "Production, Characterization, and Antioxidant Activity of Fucoxanthin from the Marine Diatom Odontella aurita", Marine Drugs, vol. 11, no. 7, pages 2667-2681, describes a composition which comprises a microalga extract, wherein the microalga may belong to the species *P. tricornutum.* Song Xia et al. also describes that the composition comprises fucoxanthin and that the microalgae extracts also contain high concentrations of fatty acids.

WO2006/077433A1 describes a composition comprising a microalgal extract comprising fucoxanthin, as well as the carotenoids diadinoxanthin, and diatoxanthin. WO2006/077433A1 also describes that the composition may be used as a pharmaceutical composition for use as a medicament, and that the extract may be prepared from *P. tricornutum.*

Sang Min Kim et al. (2012) "A Potential Commercial Source of Fucoxanthin Extracted from the Microalga", Applied Biochemistry and Biotechnology; Part A: Enzyme Engineering and Biotechnology, vol. 166, no. 7, pages 1843-1855, describes a composition comprising an extract of *P. tricornutum* which comprises fucoxanthin (15.33 mg/g), as well as other carotenoids, fatty acids and polysaccharides.

EP3116518A1 describes an extract from microalgae or microalgae biomass, which comprises fucoxanthin, fucoxanthinol, and fatty acids. EP3116518A1 also describes that the extract may comprise fatty acids and that the microalga species from which the composition is produced may be *P. tricornutum.*

Michalak et al. (2014) "Algal extracts: Technology and advances", Engineering in Life Sciences, vol. 14, no. 6, pages 581-591, describes microalgal extracts from *P. tricornutum.*

Borowitzka (2013) "High-value products from microalgae-their development and commercialisation", Journal of Applied Phycology, vol. 25, no. 3, pages 743-756, describes compositions prepared from microalgae extracts. Among the microalgal species used are *P. tricornutum* and *lsochrysis galbana.* Borowitzka (2013) also describes that several compounds may be extracted from cultured microalgae, among which carotenoids such as betacarotene and fucoxanthin, fatty acids such as PUFAs, including docosahexaenoic acid, and eicosahexaenoic acid, and polysaccharides.

WO2012/047120A1 describes a microbial biomass comprising fucoxanthin can be produced during heterotrophic growth of microbial strains and that a microbial biomass can be extracted from a microalgal biomass such as from *P. triconutum.*

Benavides et al. (2013) "Productivity and biochemical composition of P. tricornutum (Bacillariophyceae) cultures grown outdoors in tubular photobioreactors and open ponds.", Biomass and Bioenergy, vol. 54, pages 115-122, describes biomass production and the chemical composition of the diatom *P. tricornutum.*

US2015/044737 describes microalgae belonging to the Nitzschia genus, that allow high yield production of lipids, particularly of docosahexaenoic acid (DHA) and eicosapentaenoic acid (EPA), and carotenoids, especially fucoxanthin.

US2011/111038 describes a stable concentrated dietary supplement containing fucoxanthin as the main active component, wherein the supplement contains at least 10% by weight of fucoxanthin, <1 ppm iodine <1 ppm arsenic.

Kanda et al. (2014) "Extraction of Fucoxanthin from Raw Macroalgae excluding Drying and Cell Wall Disruption by Liquefied Dimethyl Ether", Marine Drugs, vol. 12, no. 5, pages 2383-2396, describes that fucoxanthin extracted from microalgae exhibits "sensitivity towards some factors such as light and pH, the least stable in acidic pH condition and higher concentration of ascorbic acid supplementation exerted stabilization role on fucoxanthin, and carotenoids are thermally decomposed in hot-drying."

### SUMMARY OF THE INVENTION

The present invention provides composition comprising: (i) a microalgae extract obtained from *Phaeodactylum tricornutum* microalgae, the microalgae extract comprising fucoxanthin and fatty acids, wherein monosaccharides and disaccharides constitute less than 0.7% by dry weight of said microalgae extract, and wherein said fucoxanthin constitutes more than 3% by dry weight of said microalgae extract; and (ii) vitamin E.

Also described herein are microalgae extract compositions exhibiting high levels of carotenoids, specifically fucoxanthin, and essential fatty acids together with low saccharide levels.

According to one aspect, there is provided a composition comprising a microalgae extract comprising: fucoxanthin and fatty acids, wherein monosaccharides and disaccharides constitute less than 0.7% by dry weight of the microalgae extract. In one embodiment, the extract comprises glucose, said glucose constitutes less than 0.1% by dry weight of the microalgae extract. In one embodiment, a ratio between the fucoxanthin and the monosaccharides and disaccharides is at least 4:1. In one embodiment, the fucoxanthin and the fatty acids constitute more than 3% and more than 30% by dry weight of said microalgae extract, respectively.

In another embodiment, the microalgae extract further comprises one or more carotenoids selected from diadinoxanthin, diatoxanthin and β-carotene, or isomers thereof.

In another embodiment, said fatty acids are selected from the group consisting of: saturated fatty acids, mono-unsaturated fatty acids, poly-unsaturated fatty acids, trans fatty acids or any combinations thereof. In another embodiment, said saturated fatty acids are one or more fatty acids selected from the group consisting of: butyric acid, caproic acid, capric acid, lauric acid, myristic acid, pentadecenoic acids, heptadecenoic acid, stearic acid, behenic acid, lignoceric acid, or isomers thereof. In another embodiment, said mono-unsaturated fatty acids are one or more fatty acids selected from the group consisting of: myristoleic acid, palmitoleic acid, oleic acid, docosenic acid, or isomers thereof. In another embodiment, said poly-unsaturated fatty acids are one or more fatty acids selected from the group consisting of: eicosapentaenic acid (EPA), linoleic acid, alpha linolenic acid, gamma linolenic acid, docosapentaenic acid, docosahexaenic acid (DHA), or isomers thereof.

According to another aspect, there is provided a composition comprising microalgae extract comprising: fucoxanthin, one or more carotenoids selected from diadinoxanthin, diatoxanthin and β-carotene or isomers thereof, palmitoleic acid, eicosapentaenic acid (EPA), archidonic acid, and docosahexaenic acid (DHA) or isomers thereof.

In another embodiment, said fucoxanthin constitutes more than 3% by dry weight of said microalgae extract. In another embodiment, said fucoxanthin constitutes more than 9% by dry weight of said microalgae extract.

In another embodiment, said palmitoleic acid constitutes more than 18% by dry weight of said microalgae extract. In another embodiment, said eicosapentaenic acid constitutes more than 20% by dry weight of said microalgae extract. In another embodiment, said archidonic acid constitutes more than 1% by dry weight of said microalgae extract. archidonic acid, said DHA constitutes more than 0.2% by dry weight of said microalgae extract.

In another embodiment, iodine constitutes less than 0.2 ppm by dry weight of said microalgae extract. In another embodiment, heavy metals constitutes less than 10 ppm by dry weight of the microalgae extract.

In another example described herein, a microalgae extract is obtained from microalgae selected from the group consisting of: *Navicula pelliculosa, Amphora, Isochrysis aff. Galbana, Odontella aurita, Nitzscia closterium, Cylindrotheca closterium, Chaetoseros sp.,* and *Emiliania huxleyi* or a combination thereof.

Also described herein is a composition comprising microalgae dried biomass comprising more than 1.6% fucoxanthin by dry weight. In another example, monosaccharides and disaccharides constitute less than 2.7% by dry weight of the microalgae dried biomass.

### BRIEF DESCRIPTION OF THE DRAWINGS

FIG. 1- High-Performance Liquid Chromatography with Diode-Array Detection (HPLC-DAD) chromatogram of *P. tricornutum* microalgae biomass recorded at 450 nm.
FIG. 2 - HPLC-DAD chromatogram of *P. tricornutum* microalgae extract recorded at 450 nm.

### DETAILED DESCRIPTION OF THE INVENTION

Herein disclosed are microalgae extract compositions comprising high levels of one or more carotenoids and/or fatty acids. Further disclosed are microalgae extract compositions comprising high levels of one or more carotenoids and/or fatty acids and low saccharide levels.

The present invention is based in part on the finding that the microalgae extracts of the invention has a unique composition which is advantageous for various fields and applications. As demonstrated hereinbelow, the microalgae extracts of the invention exhibit high fucoxanthin levels and extremely low saccharide levels.

In some embodiments, the microalgae extract or any formulation thereof may be used as a nutritional supplement, a pharmaceutical composition and/or cosmetic composition. For a non-limiting example, the microalgae extract may be incorporated in dry formulations of nutritional supplements and packaged in gel capsules, tablets, sachets and the like. In yet another example, the product may be useful in a liquid form for cosmetic preparations or packaging in soft capsules.

As used herein, the term "microalgae" means any unicellular, photosynthetic microorganism. The microalgae extract of the invention is extracted from *P. tricornutum.* In one example described herein, the microalgae extract is extracted from *Navicula pelliculosa.* In one example described herein, the microalgae extract is extracted from *Amphora.* In one example described herein, the microalgae extract is extracted from *Isochrysis aff. Galbana*. In one example described herein, the microalgae extract is extracted from *Odontella aurita*. In one example described herein, the microalgae extract is extracted from *Nitzscia closterium.* In one example described herein, the microalgae extract is example described herein from *Cylindrotheca closterium.* In one example described herein, the microalgae extract is extracted from *Chaetoseros sp.* In one example described herein, the microalgae extract is extracted from *Emiliania huxleyi.*

In one embodiment, the microalgae is a wild type microalgae. In another embodiment, the microalgae is a genetically modified microalgae.

As used herein, the microalgae extract refers to materials extracted from microalgae. In one embodiment, microalgae can be harvested prior to extraction by any conventional means including, but not limited to filtration, air flotation and centrifugation.

### Extraction methods

In one embodiment, the extraction is carried out by any means known in the art. In another embodiment, the extraction is a mechanical extraction. In another embodiment, the extraction is carried out by using an organic solvent. In one embodiment, the organic solvent is at least partially miscible in water. Non-limiting example of solvents that are miscible in water include methanol, ethanol, propanol, isopropanol, n-propanol, other alcohols containing 4 carbons or less, acetone, ketones containing 4 carbons or less, cyclic ethers such as dioxane and tetrahydrofuran, water miscible ethers such as diethyl ether, other oxygen-containing organic molecules having a ratio of carbon to oxygen atoms of about 4: 1 or less and acetonitrile, or combination thereof. In another embodiment, the organic solvent is immiscible in water. Non-limiting examples of organic solvent that are immiscible in water include alkanes such as hexane, pentane, heptane, octane, esters such as ethyl acetate, butyl acetate, ketones such as methyl ethyl ketone (MEK), methyl isobutyl ketone (MIBK), aromatics such as toluene, benzene, cyclohexane, tetrahydrofuran, haloalkanes such as chloroform, trichloroethylene and ethers such as diethyl ether, or combinations thereof.

The term "polar solvent" as used herein means a solvent that tends to interact with other compounds or itself through acid-base interactions, hydrogen bonding, dipole-dipole interactions, or by dipole-induced dipole interactions. Non-limiting examples of polar solvents include: ethanol, propylene glycol, butylene glycol, methanol, glycerol, propanol, butanol, dipropylene glycol, pentylene glycol, hexylene glycol, dimethyl formamide, acetonitrile, dimethyl sulfoxide, dichloromethane, ethyl acetate, tetrahydrofuran, formic acid, acetic acid and acetone. According to yet additional embodiments, the extraction is performed with a combination of at least two solvents.

In some embodiments, the carotenoid-containing microalgae extract, is in the form of an oleoresin, for example. The term "oleoresin" refers to a lipid extract of a carotenoid-containing material from microalgae.

In another embodiment, the extraction is carried out by using supercritical fluid-CO₂ (SCF-CO2) as known in the art. As used herein, supercritical fluid-CO₂ refer to CO2 at a temperature (e.g., 40-60° C.) and pressure above its critical point, where distinct liquid and gas phases do not exist. In one embodiment, supercritical fluid-CO₂ can effuse through solids like a gas, and dissolve materials like a liquid. In another embodiment, the extraction is carried out by using SCF-CO₂ and a co-solvent. In one embodiment, the co-solvent is selected from ethanol, acetone, methanol, and any combination thereof.

In one embodiment, an extraction by a solvent is carried out following the SCF-CO2 extraction. In one embodiment, the extraction with a solvent is a liquid-liquid extraction. In one embodiment, the solvent is a polar solvent. In one embodiment, the solvent is selected from the group consisting of: ethanol, methanol, acetone, hexane and heptane. In some embodiment, the extraction by a solvent is followed by a second extraction by a second solvent. In some embodiments, the second solvent is a polar solvent.

The term "liquid-liquid extraction", also known as solvent extraction and partitioning, refers to an extraction of a substance from one liquid into another liquid phase. In liquid-liquid extraction, substances are separated based on their relative solubilities in two different immiscible liquids (solvents), such as for a non-limiting example water and an organic solvent.

For a non-limiting example, the extraction is carried out by using supercritical fluid-CO₂ (SCF-CO2), followed by an extraction by a polar solvent, such as ethanol to enrich the ethanol extracted mass, which is followed by a second extraction with a second polar solvent (e.g., ethanol, ketone, ester, etc.).

### Microalgae extract

The microalgae extract of the invention comprises fucoxanthin in an amount of more than 3%. In one embodiment, the microalgae extract comprises fucoxanthin in an amount of more than 4%, or alternatively more than 4%, or alternatively more than 5%, or alternatively more than 6%, or alternatively more than 7%, or alternatively more than 8%, or alternatively more than 9%, or alternatively more than 10%, or alternatively more than 11%, or alternatively more than 12%, or alternatively more than 13%, or alternatively more than 14%, by dry weight. In another embodiment, the microalgae extract comprises fucoxanthin in an amount of between 3% and 15% by dry weight.

As used herein, the term dry weight (DW) refers to the weight of the dry material.

In one embodiment, the microalgae extract comprises fucoxanthin and other carotenoids. In one embodiment, the microalgae extract comprises fucoxanthin and β-carotene or isomers thereof. In one embodiment, the microalgae extract comprises fucoxanthin and diadinoxanthin or isomers thereof. In one embodiment, the microalgae extract comprises fucoxanthin and diatoxanthin or isomers thereof.

In one embodiment, the microalgae extract further comprises fatty acids. In one embodiment, the fatty acids constitute more than 40%, or alternatively more than 45%, or alternatively more than 50%, or alternatively more than 55%, or alternatively more than 60%, or alternatively more than 70%, or alternatively more than 75%, or alternatively more than 80%, or alternatively more than 85% or alternatively more than 90% or alternatively more than 95% by dry weight of the microalgae extract.

In one embodiment, the fatty acids are selected from the group consisting of: saturated fatty acids, unsaturated fatty acids, trans fatty acids and any combinations thereof.

In one embodiment, the fatty acids are selected from the group consisting of: saturated fatty acids, mono-unsaturated fatty acids, poly-unsaturated fatty acids, trans fatty acids or any combinations thereof.

As demonstrated hereinbelow, a level of the saturated fatty acids in the microalgae extract is at least 5, 6, 7 or 8 folds lower than a level the saturated fatty acids in macro-algae extracts.

The saturated fatty acids of the present disclosure may constitute more than 8%, or alternatively more than 9%, or alternatively more than 10%, or alternatively more than 11%, or alternatively more than 12%, or alternatively more than 13%, or alternatively more than 14%, or alternatively more than 15%, or alternatively more than 16% by dry weight of the microalgae extract. In one embodiment, the saturated fatty acids constitute less than 8%, or alternatively less than 9%, or alternatively less than 10%, or alternatively less than 11%, or alternatively less than 12%, or alternatively less than 13%, or alternatively less than 14%, or alternatively less than 15%, or alternatively less than 16%, or alternatively less than 20%, or alternatively less than 25% by dry weight of the microalgae extract. In one embodiment, the saturated fatty acids constitute less than 10% by dry weight of the microalgae extract. In one embodiment, the saturated fatty acids constitute less than 15% by dry weight of the microalgae extract. In one embodiment, the saturated fatty acids constitute between 5-20%, or alternatively between 5-18%, or alternatively between 6-18%, or alternatively between 7-18%, or alternatively between 5-15%, or alternatively between 5-10% by dry weight of the microalgae extract.

In one embodiment, the unsaturated fatty acids constitute more than 30%, or alternatively more than 35%, or alternatively more than 40%, or alternatively more than 45%, or alternatively more than 46%, or alternatively more than 50%, or alternatively more than 54%, or alternatively more than 55%, or alternatively more than 56% by dry weight of the microalgae extract. Each possibility represents a separate embodiment of the present invention. In one embodiment, the unsaturated fatty acids constitute between 40-70%, or alternatively between 45-60%, or alternatively between 50-70%, or alternatively between 50-65%, or alternatively between 50-60%, or alternatively between 55-65% by dry weight of the microalgae extract.

In one embodiment, the poly-unsaturated fatty acids constitute more than 15%, or alternatively more than 16%, or alternatively more than 17%, or alternatively more than 18%, or alternatively more than 19%, or alternatively more than 20%, or alternatively more than 21%, or alternatively more than 22%, or alternatively more than 23%, or alternatively more than 24% , or alternatively more than 25% , or alternatively more than 26%, or alternatively more than 27% , or alternatively more than 28%, or alternatively more than 29%, or alternatively more than 30%, or alternatively more than 31%, or alternatively more than 32% by dry weight of the microalgae extract. In one embodiment, the poly-unsaturated fatty acids constitute more than 20% by dry weight of the microalgae extract. In one embodiment, the poly-unsaturated fatty acids constitute more than 25% by dry weight of the microalgae extract. In one embodiment, the poly-unsaturated fatty acids constitute between 15-50%, or alternatively between 15-40%, or alternatively between 20-40%, or alternatively between 25-40%, or alternatively between 20-35%, or alternatively between 25-35% by dry weight of the microalgae extract.

In one embodiment, the mono-unsaturated fatty acids constitute more than 10%, or alternatively more than 11%, or alternatively more than 12%, or alternatively more than 13%, or alternatively more than 14%, or alternatively more than 15%, or alternatively more than 16%, or alternatively more than 17%, or alternatively more than 18%, or alternatively more than 19%, or alternatively more than 20% by dry weight of the microalgae extract. In one embodiment, the mono-unsaturated fatty acids constitute more than 15% by dry weight of the microalgae extract. In one embodiment, the mono-unsaturated fatty acids constitute more than 18% by dry weight of the microalgae extract. In one embodiment, the mono-unsaturated fatty acids constitute between 10-30%, or alternatively between 12-30%, or alternatively between 15-30%, or alternatively between 10-28%, or alternatively between 10-25%, or alternatively between 15-28%, or alternatively between 15-25% by dry weight of the microalgae extract.

In one embodiment, the trans fatty acids constitute more than 3%, or alternatively more than 3.5%, or alternatively more than 4%, or alternatively more than 4.5%, or alternatively more than 5%, or alternatively more than 5.5%, or alternatively more than 6%, or alternatively more than 6.5% by dry weight of the microalgae extract. In one embodiment, the trans fatty acids constitute more than 5% by dry weight of the microalgae extract. In one embodiment, the trans fatty acids constitute more than 6% by dry weight of the microalgae extract. In one embodiment, the trans fatty acids constitute between 3-15%, or alternatively between 4-15%, or alternatively between 3-10%, or alternatively between 3-9%, or alternatively between 4-10%, or alternatively between 4-9%, or alternatively between 5-9% by dry weight of the microalgae extract.

In one embodiment, the saturated fatty acids are one or more fatty acids selected from the group consisting of: butyric acid, caproic acid, capric acid, lauric acid, myristic acid, pentadecenoic acids, palmitic acid (PA), heptadecenoic acid, stearic acid, behenic acid, lignoceric acid, or isomers thereof.

In one embodiment, the mono-unsaturated fatty acids are one or more fatty acids selected from the group consisting of: myristoleic acid, palmitoleic acid, oleic acid, docosenic acid, or isomers thereof.

In one embodiment, the poly-unsaturated fatty acids are one or more fatty acids selected from the group consisting of: eicosapentaenic acid (EPA), linoleic acid, alpha linolenic acid, gamma linolenic acid, docosapentaenic acid, docosahexaenic acid (DHA), or isomers thereof.

In one embodiment, the microalgae extract further comprises palmitoleic acid or isomers thereof, wherein the palmitoleic acid constitutes more than 5%, or alternatively more than 8%, or alternatively more than 10%, or alternatively more than 11%, or alternatively more than 12%, or alternatively more than 13%, or alternatively more than 14%, or alternatively more than 15%, or alternatively more than 16%, or alternatively more than 17%, or alternatively more than 18%, or alternatively more than 19%, or alternatively more than 20%, or alternatively more than 21%, or alternatively more than 22% by dry weight of the microalgae extract.

In one embodiment, the microalgae extract further comprises eicosapentaenic acid (EPA) or isomers thereof, wherein said eicosapentaenic acid constitutes more than 1.5% or alternatively more than 2%, or alternatively more than 3%, or alternatively more than 4%, or alternatively more than 5%, or alternatively more than 6%, or alternatively more than 7%, or alternatively more than 7.5%, or alternatively more than 10%, or alternatively more than 11%, or alternatively more than 12%, or alternatively more than 13%, or alternatively more than 14%, or alternatively more than 15%, or alternatively more than 16%, or alternatively more than 17%, or alternatively more than 18%, or alternatively more than 19%, or alternatively more than 20%, or alternatively more than 21%, or alternatively more than 22%, or alternatively more than 23%, or alternatively more than 24%, or alternatively more than 25%, by dry weight of the microalgae extract.

In one embodiment, the microalgae extract further comprises archidonic acid (AA) or isomers thereof, wherein said AA constitute more than 0.1% or alternatively more than 0.2%, or alternatively more than 0.5%, or alternatively more than 0.6%, or alternatively more than 0.7%, or alternatively more than 0.9%, or alternatively more than 1%, or alternatively more than 1.5%, or alternatively more than 2%, or alternatively more than 2.5%, or alternatively more than 3% by dry weight of the microalgae extract.

In one embodiment, the microalgae extract further comprises docosahexaenic acid (DHA) or isomers thereof, wherein said DHA constitute more than 0.1% or alternatively more than 0.15%, or alternatively more than 0.2%, or alternatively more than 0.3% by dry weight of the microalgae extract.

In one embodiment, the microalgae extract further comprises palmitic acid (PA) or isomers thereof, wherein said PA constitute more than 5% or alternatively more than 6%, or alternatively more than 7%, or alternatively more than 8%, or alternatively more than 8.5% by dry weight of the microalgae extract.

In one embodiment, the microalgae extract comprises: fucoxanthin and fatty acids. In some embodiments, the weight to weight ratio of the fucoxanthin to the fatty acids in the extract ranges between 1:10 and 1:30. In some embodiments, the weight to weight ratio of the fucoxanthin to the fatty acids ranges between 1:10 and 1:20.

In some embodiments, the extract comprises fucoxanthin and unsaturated fatty acids. In some embodiments, the weight to weight ratio of the fucoxanthin to the unsaturated fatty acids in the extract ranges between 1:5 and 1:30, 1:5 and 1:20, 1:10 and 1:30 or 1:10 and 1:20. In some embodiments, the unsaturated fatty acids comprises monounsaturated fatty acids and polyunsaturated fatty acids.

In some embodiments, the weight to weight ratio of the fucoxanthin to the mono and poly unsaturated fatty acids in the extract ranges between 1:5 and 1:30, 1:5 and 1:20, 1:10 and 1:30 or 1:10 and 1:20. In some embodiments, the weight to weight ratio of the fucoxanthin to the poly-unsaturated fatty acids of the extract ranges between 1:3 and 1:30, 1:3 and 1:20, 1:3 to 1:15, 1:3 to 1:10, 1:4 and 1:30, 1:4 and 1:20, 1:4 to 1:15, 1:4 to 1:10, 1:5 and 1:30, 1:5 and 1:20, 1:5 to 1:15 or 1:5 and 1:10. In some embodiments, the weight to weight ratio of the fucoxanthin to the mono-unsaturated fatty acids of the extract ranges between 1:3 and 1:30, 1:3 and 1:20, 1:3 to 1:15, 1:3 to 1:10, 1:4 and 1:30, 1:4 and 1:20, 1:4 to 1:15, 1:4 to 1:10, 1:5 and 1:30, 1:5 and 1:20, 1:5 to 1:15 or 1:5 and 1:10.

The present disclosure also provides a composition comprising microalgae extract comprising: fucoxanthin, palmitoleic acid, eicosapentaenic acid (EPA), archidonic acid (AA), gamma linolenic acid, docosahexaenic acid (DHA) and palmitic acid (PA) or isomers thereof.

The present disclosure also provides a composition comprising microalgae extract comprising: fucoxanthin, one or more carotenoids selected from diadinoxanthin, diatoxanthin and β-carotene or isomers thereof, palmitoleic acid, eicosapentaenic acid (EPA), archidonic acid (AA), gamma linolenic acid, docosahexaenic acid (DHA) and palmitic acid (PA) or isomers thereof.

In one embodiment, the fucoxanthin constitutes more than 3%, or alternatively more than 4%, or alternatively more than 5%, or alternatively more than 6%, or alternatively more than 7%, or alternatively more than 8%, or alternatively more than 9%, or alternatively more than 10%, or alternatively more than 11%, or alternatively more than 12%, or alternatively more than 13%, or alternatively more than 14%, by dry weight of said microalgae extract.

In one embodiment, the palmitoleic acid and/or isomers thereof constitute more than 5%, or alternatively more than 8%, or alternatively more than 10%, or alternatively more than 11%, or alternatively more than 12%, or alternatively more than 13%, or alternatively more than 14%, or alternatively more than 15%, or alternatively more than 16%, or alternatively more than 17%, or alternatively more than 18%, or alternatively more than 19%, or alternatively more than 20%, or alternatively more than 21%, or alternatively more than 22% by dry weight of the microalgae extract.

In one embodiment, the microalgae extract comprises: the fucoxanthin and the palmitoleic acid and/or isomers thereof. In some embodiments, the weight to weight ratio of the fucoxanthin to the palmitoleic acid in the extract ranges between 2:1 and 1:10, 2:1 and 1:5, 2:1 and 1:2, 1:1 and 1:10, 1:1 and 1:5, 1:1 and 1:2, 1:2 and 1:10, or 1:2 and 1:5.

In one embodiment, the eicosapentaenic acid (EPA) and/or isomers thereof constitute more than 1.5% or alternatively more than 2%, or alternatively more than 3%, or alternatively more than 4%, or alternatively more than 5%, or alternatively more than 6%, or alternatively more than 7%, or alternatively more than 8%, or alternatively more than 10%, or alternatively more than 11%, or alternatively more than 12%, or alternatively more than 13%, or alternatively more than 14%, or alternatively more than 15%, or alternatively more than 16%, or alternatively more than 17%, or alternatively more than 18%, or alternatively more than 19%, or alternatively more than 20%, or alternatively more than 21%, or alternatively more than 22%, or alternatively more than 23%, or alternatively more than 24%, or alternatively more than 25% by dry weight of the microalgae extract.

In one embodiment, the microalgae extract comprises: the fucoxanthin and the EPA. In some embodiments, the weight to weight ratio of the fucoxanthin to the EPA in the extract ranges between 2:1 and 1:10, 2:1 and 1:5, 2:1 and 1:2, 1:1 and 1:10, 1:1 and 1:5, 1:1 and 1:2, 1:2 and 1:10, 1:2 and 1:8, 1:2 and 1:7, or 1:2 and 1:6. Each possibility represents a separate embodiment of the present invention. In some embodiments, the weight to weight ratio of the fucoxanthin to the EPA in the extract ranges between 1:2 and 1:8.

In one embodiment, the archidonic acid (AA) and/or isomers thereof constitute more than 0.1% or alternatively more than 0.2%, or alternatively more than 0.5%, or alternatively more than 0.6%, or alternatively more than 0.7%, or alternatively more than 0.9%, or alternatively more than 1%, or alternatively more than 1.5%, or alternatively more than 2%, or alternatively more than 2.5%, or alternatively more than 3% by dry weight of the microalgae extract.

In some embodiments, the extract comprises fucoxanthin and archidonic acid (AA) and/or isomers thereof. In some embodiments, the weight to weight ratio of the fucoxanthin to the AA in the extract ranges between 4:1 and 1:2, 3:1 and 1:2, 2:1 and 1:2, 1:1 and 1:2, 1.5:1 and 1:1.5, 4:1 and 1:1, 3:1 and 1:1, 2:1 and 1:1, or 1.5:1 and 1:1. In some embodiments, the weight to weight ratio of the fucoxanthin to the AA in the extract ranges between 2:1 and 1:1. In some embodiments, the weight to weight ratio of the fucoxanthin to the AA in the extract ranges between 1.5:1 and 1:1.

In one embodiment, DHA and/or isomers thereof constitute more than 0.1% or alternatively more than 0.15%, or alternatively more than 0.2%, or alternatively more than 0.24%, or alternatively more than 0.3% by dry weight of the microalgae extract.

In one embodiment, the microalgae extract comprises: fucoxanthin and DHA. In some embodiments, the weight to weight ratio of the fucoxanthin to the DHA in the extract ranges between 10:1 and 1:1, 8:1 and 1:1, 7:1 and 1:1, 6:1 and 1:1, 5:1 and 1:1, 4:1 and 1:1, 10:1 and 2:1, 8:1 and 2:1, 7:1 and 2:1, 6:1 and 2:1, 5:1 and 2:1, 4:1 and 2:1, 10:1 and 3:1, 8:1 and 3:1, 7:1 and 3:1, 6:1 and 3:1, 5:1 and 3:1, or 4:1 and 3:1. In some embodiments, the weight to weight ratio of the fucoxanthin to the DHA in the extract ranges between 6:1 and 2:1. In some embodiments, the weight to weight ratio of the fucoxanthin to the DHA in the extract ranges between 5:1 and 3:1.

In one embodiment, the PA and/or isomers thereof constitute more than 5% or alternatively more than 6%, or alternatively more than 7%, or alternatively more than 8%, or alternatively more than 8.5% by dry weight of the microalgae extract.

In one embodiment, the microalgae extract comprises: fucoxanthin and PA. In some embodiments, the weight to weight ratio of the fucoxanthin to the PA in the extract ranges between 2:1 and 1:10, 2:1 and 1:8, 2:1 and 1:7, 2:1 and 1:6, 2:1 and 1:5, 2:1 and 1:4, 1:1 and 1:10, 1:1 and 1:8, 1:1 and 1:7, 1:1 and 1:6, 1:1 and 1:5, 1:1 and 1:4, 1:2 and 1:10, 1:2 and 1:8, 1:2 and 1:7, 1:2 and 1:6, 1:2 and 1:5, 1:2 and 1:4, 1:3 and 1:10, 1:3 and 1:8, 1:3 and 1:7, 1:3 and 1:6, or 1:3 and 1:5. In some embodiments, the weight to weight ratio of the fucoxanthin to the PA in the extract ranges between 1:3 and 1:5. In some embodiments, the weight to weight ratio of the fucoxanthin to the PA in the extract ranges between 1:2 and 1:6.

In one embodiment of the invention, iodine constitutes less than 0.2 parts per million (ppm) by dry weight of the microalgae extract. In one embodiment of the invention, iodine constitutes less than 0.5 parts per million (ppm) by dry weight of the microalgae extract.

In one embodiment of the invention, heavy metals (e.g., mercury, lead, cadmium, arsenic etc.) constitute less than 10 ppm or less than 5 ppm by dry weight of the microalgae extract.

In some embodiment of the invention, monosaccharides and disaccharides constitute less than 0.7%, 0.6%, 0.5%, 0.4%, 0.3%, 0.2%, 0.1%, 0.05% of the microalgae extract. In some embodiment of the invention, monosaccharides and disaccharides constitute less than 0.1% of the microalgae extract. In one embodiment, the microalgae extract is substantially free of monosaccharides and disaccharides. In some embodiments, a microalgae extract substantially free of monosaccharides and disaccharides comprises 0.7%, 0.6%, 0.5%, 0.4%, 0.3%, 0.2%, 0.1% or less monosaccharides and disaccharides by dry weight. . In some embodiments, a microalgae extract substantially free of monosaccharides and disaccharides comprises 0.7% or less monosaccharides and disaccharides by dry weight. In some embodiments, a microalgae extract substantially free of monosaccharides and disaccharides comprises 0.1% or less monosaccharides and disaccharides by dry weight. In some embodiments, the weight to weight ratio of the fucoxanthin to the monosaccharides and disaccharides is at least 4:1, at least 5:1, at least 7:1, at least 10:1, or at least 20:1.

In some embodiment, glucose constitutes less than 0.7%, 0.6%, 0.5%, 0.4%, 0.3%, 0.2%, 0.1%, 0.05% of the microalgae extract. In some embodiment, glucose constitutes less than 0.1% of the microalgae extract. In one embodiment, the microalgae extract is substantially free of glucose. In some embodiments, a microalgae extract substantially free of glucose comprises 0.5%, 0.4%, 0.3%, 0.2%, 0.1% or less glucose by dry weight. In some embodiments, a microalgae extract substantially free of glucose comprises 0.1% or less glucose by dry weight.

In some embodiments, the weight to weight ratio of the fucoxanthin to the glucose is at least 5:1, at least 7:1, at least 10:1, at least 20:1, at least 30:1, at least 40:1, or at least 50:1. Each possibility represents a separate embodiment of the present invention. In some embodiments, the weight to weight ratio of fucoxanthin to glucose, in the extract, ranges between 10:1 and 100:1, 10:1 and 50:1, 10:1 and 40:1, 10:1 and 30:1, 20:1 and 100:1, 20:1 and 50:1, 20:1 and 40:1, or 20:1 and 30:1. In some embodiments, the weight to weight ratio of fucoxanthin to glucose, in the extract, ranges between 20:1 and 40:1. In some embodiments, the weight to weight ratio of fucoxanthin to glucose, in the extract, is at least 20:1.

In some embodiment, sugar constitutes less than 1%, 0.9%, 0.8%, 0.7%, 0.6%, 0.5%, 0.4%, 0.3%, 0.2%, 0.1%, 0.05% of the microalgae extract. In some embodiments, the weight to weight ratio of the fucoxanthin to the sugar ranges between 2:1 and 10:1, 2.5:1 and 10:1, 3:1 and 10:1, 4:1 and 10:1, or 5:1 and 10:1. In some embodiments, the weight to weight ratio of fucoxanthin to saccharides is at least 2:1, 2.5:1, 3:1, 4:1, 5:1, or 10:1.

In some embodiment, saccharides constitute less than 2%, 1.5%, 1%, 0.9%, 0.8%, 0.7%, 0.6%, 0.5%, 0.4%, 0.3%, 0.2%, 0.1%, 0.05% of the microalgae extract.

In some embodiments, the weight to weight ratio of fucoxanthin to saccarides, in the extract, ranges between 2:1 and 10:1, 2.5:1 and 10:1, 3:1 and 10:1, 4:1 and 10:1, or 5:1 and 10:1. In some embodiments, the weight to weight ratio of fucoxanthin to saccharides is at least 2:1, 2.5:1, 3:1, 4:1, 5:1, or 10:1.

The term "saccharide" refers to a carbohydrate which is a polyhydroxy aldehyde or ketone, or derivative thereof. As used herein, the term "saccharide" encompasses monosaccharides, disaccharides, oligosaccharides and polysaccharides, or derivatives thereof. Monosaccharides, or simple sugars, consist of a single polyhydroxy aldehyde or ketone unit. As used herein, the term "monosaccharide" refers to the basic unit of carbohydrates. Non-limiting examples of monosaccharides include: mannose, glucose (dextrose), fructose, galactose, xylose, and ribose. The term "glucose" refers to a monosaccharide having the chemical formula, C₆H₁₂O₆, which is also known as D-glucose or dextrose. As used herein, the term "disaccharide" refers to carbohydrates composed of two monosaccharides. Non-limiting examples of disaccharides include: sucrose, lactose and maltose. Oligosaccharides typically contain from 2 to 10 monosaccharide units joined in glycosidic linkage. Polysaccharides (glycans) typically contain more than 10 such units. The term "sugar" generally refers to mono-, di- or oligosaccharides.

The present disclosure also provides a composition comprising microalgae extract comprising fucoxanthin, wherein the extract is substantially free of monosaccharides and disaccharides. In one embodiment, the invention provides a composition comprising microalgae extract comprising: fucoxanthin and fatty acids, wherein the extract is substantially free of monosaccharides and disaccharides. In one embodiment, the invention provides a composition comprising microalgae extract comprising: fucoxanthin and fatty acids, wherein monosaccharides and disaccharides constitute less than 0.1% by dry weight of the microalgae extract. In one embodiment, the invention provides a composition comprising microalgae extract comprising: fucoxanthin, palmitoleic acid, eicosapentaenic acid (EPA), archidonic acid (AA), gamma linolenic acid, docosahexaenic acid (DHA) and palmitic acid (PA) or isomers thereof, wherein the extract is substantially free of monosaccharides and disaccharides. In one embodiment, the invention provides a composition comprising microalgae extract comprising: fucoxanthin, one or more carotenoids selected from diadinoxanthin, diatoxanthin and β-carotene or isomers thereof, palmitoleic acid, eicosapentaenic acid (EPA), archidonic acid (AA), gamma linolenic acid, docosahexaenic acid (DHA) and palmitic acid (PA) or isomers thereof, wherein the extract is substantially free of monosaccharides and disaccharides.

### Microalgae production

In one embodiment, the microalgae are grown in a defined culture medium. A suitable culture medium is any medium known in the art that support the viability and growth of the microalgae. In one embodiment, the culture medium comprises a nitrogen source selected from the group consisting: nitrate (NO₃), ammonium (NH₄⁺) and urea (CH₄N₂O) or a combination thereof. In one embodiment, the culture medium comprises urea. In one embodiment, the medium comprises less than about 0.5 gram/liter urea. In one embodiment, the medium comprises between 0.3 gram/liter to 0.8 gram/ liter urea. In one embodiment, the medium comprises between 0.5 gram/liter to 1 gram/ liter urea. In one embodiment, the medium comprises between 1 gram/liter to 2 gram/ liter urea. In one embodiment, the medium comprises between 0.5 gram/liter to 3 gram/ liter urea. In one embodiment, the microalgae use the urea as a nitrogen source. In one embodiment, the microalgae use the urea as a sole source of nitrogen.

In one embodiment, the culture medium comprises phosphate. In one embodiment, the medium comprises less than 0.1 gram/liter phosphate. In one embodiment, the medium comprises between 0.05 to 0.5 gram/liter phosphate. In one embodiment, the medium comprises between 0.5 to 2 gram/liter phosphate. In one embodiment, the medium comprises more than 2 gram/liter phosphate.

In one embodiment, the culture medium comprises a salt selected from the group consisting: sodium chloride (NaCl), Magnesium Sulfate (MgSO4), Magnesium Chloride (MgCl2), Calcium Chloride (CaCl2) or a combination thereof. In one embodiment, the medium comprises less than 5-30 gram/liter NaCl. In one embodiment, the medium comprises between 8 to 27 gram/liter NaCl. In one embodiment, the medium comprises between 1 to 5 gram/liter NaCl. In one embodiment, the medium comprises between 5 to 10 gram/liter NaCl. In one embodiment, the medium comprises less than 27 gram/liter NaCl.

In one embodiment, the medium is substantially free of silica. As used herein a medium substantially free of silica comprises less than 0.01 gram/liter silica, or alternatively less than 0.05 gram/liter silica, or alternatively less than 0.1 gram/liter silica, or alternatively less than 0.5 gram/liter silica.

### Microalgae biomass

In an alternative aspect, there is provided microalgae biomass. The term "biomass" refers to any living or recently dead biological cellular material derived from microalgae. In one embodiment, the microalgae biomass is obtained from microalgae cell culture. In one embodiment, the microalgae biomass is a harvested biomass. In one embodiment, the microalgae biomass is a dried product of microalgae cells.

A person skilled in the art will appreciate that, the biomass may be harvested by any conventional means including, but not limited to filtration, air flotation and centrifugation. Additionally, dried biomass may be produced by various process known in the art. Non-limiting examples of drying techniques which are commonly used include: drum drying, rotary drying, freeze drying, solar drying, and spray drying.

As used herein, "Drum drying" refers to a method used for drying out microalgae into a film or paste using a large rotating drum that slowly applies heat. "Rotary drying" is much like drum drying except that an air pump is used to alter the pressure in order to evaporate water. "Freeze drying" refers to a dehydration process which works by freezing the subject material and then reducing the surrounding pressure and adding enough heat to allow the frozen water in the material to sublime directly from the solid phase to the gas phase. "Solar drying" refers to a method which uses glass and lenses to focus and trap heat from the sun. "Spray drying" refers to a method of producing a dry powder from a liquid or slurry by rapidly drying with a hot gas.

In some embodiments, one or more stabilizers are added to the biomass prior to obtaining a dried biomass in order to stabilize the Fucoxanthin content of the biomass. In some embodiments the stabilizers are antioxidants. In some embodiments the stabilizers are lipophilic antioxidants. Non-limiting examples of antioxidants include: vitamin C, Ascorbyl palmitate, vitamin E, and rosemary oil.

In some embodiments, stabilizers are added to the biomass, such that the stabilizer constitute between 0.1% and 5% by weight of the biomass prior to drying.

In some embodiments, the stabilizers are added following the extraction process. In some embodiments, stabilizers are added to the microalgae extract, such that the stabilizer constitute between 0.1% and 5% by weight of the microalgae extract. For a non-limiting example, Vitamin E and/or Ascorbyl palmitate, which are both lipophilic materials, may be added to the microalgae extract.

Cheol-Ho Pan et al. (Appl Biochem Biotechnol (2012) 166:1843-1855) disclosed 1.533% fucoxanthin by dry weight of P. tricornutum extract. Notably, this was achieved by cultivating the microalgae in 30 Liter plastic cylinders. Attempts to reach high fucoxanthin contents using biomass production techniques (e.g., using photobioreactors) resulted in substantially lower fucoxanthin contents. As such, Guil-Guerrero (Journal of Food Biochemisby 25, 2001, 57-76) reported microalgae biomass production reaching less than 0.45% carotenoids having about 50% fucoxanthin content.

Described herein in some examples, a composition comprising microalgae dried biomass comprising more than 0.5%, more than 0.6%, more than 0.7%, more than 0.8%, more than 0.9%, more than 1%, more than 1.1%, more than 1.2%, more than 1.3%, more than 1.5%, more than 1.6%, more than 1.7%, more than 1.8%, more than 1.9%, more than 2%, more than 2.1%, more than 2.2%, more than 2.3%, more than 2.4% or more than 2.5% fucoxanthin by dry weight, said microalgae is cultured in a photobioreactor.

As used herein, the term "photobioreactor" refers to a device or system used to support a biologically active environment for the mass (e.g., above 100 Liter) cultivation and/or production of microorganisms capable of performing photosynthesis, such as microalgae. The photobioreactor supplies a specifically controlled environment, allowing utilization of a light source (e.g., sun light) for autotrophic growth of the microorganisms. Autotrophic growth refers to the capability of an organism to synthesize its own food from inorganic substances, using light or chemical energy.

In one example, described herein is a composition comprising microalgae dried biomass comprising more than 1% fucoxanthin by dry weight. In one example, described herein is a composition comprising microalgae dried biomass comprising more than 1.1%, or alternatively more than 1.2%, or alternatively more than 1.3%, or alternatively more than 1.4%, or alternatively more than 1.5%, or alternatively more than 1.6%, or alternatively more than 1.7%, or alternatively more than 1.8%, or alternatively more than 1.9%, or alternatively more than 2% fucoxanthin by dry weight.

In one example, fucoxanthin constitutes at least 1%, or alternatively at least 1.2%, at least 1.3%, at least 1.4%, at least 1.5%, at least 1.6%, at least 1.7%, at least 1.8%, at least 1.9%, at least 2% by dry weight of the microalgae dried biomass.

In one embodiment, the microalgae dried biomass comprises fucoxanthin and other carotenoids. In one embodiment, the microalgae dried biomass comprises fucoxanthin and β-carotene or isomers thereof. In one embodiment, the microalgae dried biomass comprises fucoxanthin and diadinoxanthin or isomers thereof. In one embodiment, the microalgae dried biomass comprises fucoxanthin and diatoxanthin or isomers thereof.

In one embodiment, the microalgae dried biomass further comprises fatty acids.

In one embodiment, the fatty acids constitutes more than 5%, or alternatively more than 6%, or alternatively more than 7%, or alternatively more than 8%, or alternatively more than 9%, or alternatively more than 10%, or alternatively more than 11%, or alternatively more than 12%, or alternatively more than 13%, or alternatively more than 14% by dry weight of the microalgae dried biomass.

In one embodiment, the saturated fatty acids constitute more than 4%, or alternatively more than 5%, or alternatively more than 6%, or alternatively more than 7%, or alternatively more than 8%, or alternatively more than 9% by dry weight of the microalgae dried biomass. In one embodiment, the saturated fatty acids constitute less than 4%, or alternatively less than 5%, or alternatively more than 6%, or alternatively less than 7%, or alternatively less than 8%, or alternatively less than 9% by dry weight of the microalgae dried biomass. In one embodiment, the saturated fatty acids constitute between 2 and 10%, 3 and 10%, 4 and 10%, 5 and 10%, 2 and 8%, 3 and 8%, 2 and 6%, or 3 and 6% by dry weight of the microalgae dried biomass.

In one embodiment, the unsaturated fatty acids constitute more than 4%, or alternatively more than 5%, or alternatively more than 6%, or alternatively more than 7%, or alternatively more than 8%, or alternatively more than 9% by dry weight of the microalgae dried biomass. In one embodiment, the unsaturated fatty acids constitute between 4% and 20%, between 4% and 15%, between 4% and 10%, between 5% and 20%, between 5% and 15%, between 5% and 10%, between 6% and 20%, between 6% and 15%, between 6% and 10%, between 7% and 20%, between 7% and 15%, or between 7% and 10 by dry weight of the microalgae dried biomass.

In one embodiment, the poly-unsaturated fatty acids constitute more than 1%, or alternatively more than 2%, or alternatively more than 3%, or alternatively more than 3.5%, or alternatively more than 4%, or alternatively more than 4.5% by dry weight of the microalgae dried biomass. In one embodiment, the poly-unsaturated fatty acids constitute between 1% and 10%, 1% and 7%, 1% and 6%, 1% and 5%, 2% and 10%, 2% and 7%, 2% and 6%, 2% and 5%, 3% and 10%, 3% and 7%, 3% and 6%, 3% and 5% by dry weight of the microalgae dried biomass. . In one embodiment, the poly-unsaturated fatty acids constitute between 3% and 5% by dry weight of the microalgae dried biomass.
In one embodiment, the mono-unsaturated fatty acids constitute more than 0.5%, or alternatively more than 1%, or alternatively more than 1.5%, or alternatively more than 2%, or alternatively more than 3%, or alternatively more than 3.5%, or alternatively more than 4%, or alternatively more than 4.5% by dry weight of the microalgae dried biomass. In one embodiment, the mono-unsaturated fatty acids constitute between 1% and 10%, 1% and 7%, 1% and 6%, 1% and 5%, 2% and 10%, 2% and 7%, 2% and 6%, 2% and 5%, 3% and 10%, 3% and 7%, 3% and 6%, or 3% and 5% by dry weight of the microalgae dried biomass. In one embodiment, the mono-unsaturated fatty acids constitute between 3% and 5% by dry weight of the microalgae dried biomass.

In one embodiment, the trans fatty acids constitute more than 0.4%, or alternatively more than 0.5%, or alternatively more than 0.6%, or alternatively more than 0.7%, or alternatively more than 1%, or alternatively more than 1.5%, or alternatively more than 2%, or alternatively more than 2.5%, or alternatively more than 3% by dry weight of the microalgae dried biomass. In one embodiment, the trans fatty acids constitute between 0.4% and 3%, 0.4% and 2%, 0.4% and 1.5%, 0.4% and 1%, 0.5% and 3%, 0.5% and 2%, 0.5% and 1.5%, or 0.5% and 1% by dry weight of the microalgae dried biomass. In one embodiment, the trans fatty acids constitute between 0.5% and 1% by dry weight of the microalgae dried biomass.

In one embodiment, the microalgae dried biomass further comprises one or more carotenoids selected from diadinoxanthin, diatoxanthin and β-carotene or isomers thereof.

In one embodiment, the microalgae dried biomass comprising fucoxanthin further comprises palmitoleic acid and/or isomers thereof. In one embodiment, the palmitoleic acid and/or isomers thereof constitute more than 1.5%, or alternatively more than 2%, or alternatively more than 2.5%, or alternatively more than 3% by dry weight of the microalgae dried biomass.

In one embodiment, the microalgae dried biomass comprising fucoxanthin further comprises eicosapentaenic acid and/or isomers thereof. In one embodiment, the eicosapentaenic acid and/or isomers thereof constitute more than 1% or alternatively more than 1.5%, or alternatively more than 2%, or alternatively more than 3%, or alternatively more than 3.5%, or alternatively more than 3.6%, or alternatively more than 3.7%, or alternatively more than 4% by dry weight of the microalgae dried biomass.

In one embodiment, the microalgae dried biomass comprising fucoxanthin further comprises archionic acid and/or isomers thereof. In one embodiment, the AA and/or isomers thereof constitute more than 0.1% or alternatively more than 0.01%, or alternatively more than 0.02%, or alternatively more than 0.03%, or alternatively more than 0.04%, or alternatively more than 0.05%, or alternatively at least 0.06 by dry weight of the microalgae dried biomass.

In one embodiment, the microalgae dried biomass comprising fucoxanthin further comprises archidonic acid (AA) and/or isomers thereof. In one embodiment, the archionic acid and/or isomers thereof constitute about 0.2% - 0.5%, or alternatively more than 0.2%- 0.4%, or alternatively about 0.3%, by dry weight of the microalgae dried biomass.

In one embodiment, the microalgae dried biomass comprising fucoxanthin further comprises DHA and/ or isomers thereof. In one embodiment, DHA and/ or isomers thereof constitute more than 0.05%, or alternatively more than 0.9%, or alternatively more than 0.10%, or alternatively more than 0.11%, or alternatively more than 0.12%, or alternatively more than 0.13%, or alternatively more than 0.14%, or alternatively more than 0.15%, or alternatively more than 0.16% by dry weight of the microalgae dried biomass.

In one embodiment, the microalgae dried biomass comprising fucoxanthin further comprises PA and/ or isomers thereof. In one embodiment, the PA and/ or isomers thereof constitute more than 1% or alternatively more than 1.1%, or alternatively more than 1.2%, or alternatively more than 1.3%, or alternatively more than 1.4%, or alternatively at least 1.5%, or alternatively at least 2%, or alternatively at least 3%, by dry weight of the microalgae dried biomass.

In another embodiment, the invention provides a composition comprising microalgae dried biomass comprising: fucoxanthin, one or more carotenoids selected from diadinoxanthin, diatoxanthin and β-carotene or isomers thereof, palmitoleic acid, eicosapentaenic acid (EPA), archidonic acid (AA), gamma linolenic acid, docosahexaenic acid (DHA) and palmitic acid (PA) or isomers thereof.

In one example, the microalgae dried biomass comprises less than 5%, 4.5%, 4%, 3.5%, 3%, 2.9%, 2.8%, 2.7%, 2.6% monosaccharides and disaccharides.

In one example, the microalgae dried biomass comprises less than 5%, 4.5%, 4%, 3.5%, 3%, 2.9%, 2.8%, 2.7%, 2.6% glucose. In one example, the microalgae dried biomass comprises less than 2.7% glucose.

In one example, the microalgae dried biomass comprises less than 5%, 4.5%, 4%, 3.5%, 3%, 2.9%, 2.8%, 2.7%, 2.6% sugars.

In one embodiment of the invention, iodine constitutes less than 0.3 ppm by dry weight of the microalgae dried biomass. In one embodiment of the invention, heavy metals (e.g., mercury, led, cadmium, arsenic, etc.) constitute less than 0.5 ppm by dry weight of the microalgae dried biomass.

In the discussion unless otherwise stated, adjectives such as "substantially" and "about" modifying a condition or relationship characteristic of a feature or features of an embodiment of the invention, are understood to mean that the condition or characteristic is defined to within tolerances that are acceptable for operation of the embodiment for an application for which it is intended. Unless otherwise indicated, the word "or" in the specification and claims is considered to be the inclusive "or" rather than the exclusive or, and indicates at least one of, or any combination of items it conjoins.

In the description and claims of the present application, each of the verbs, "comprise," "include" and "have" and conjugates thereof, are used to indicate that the object or objects of the verb are not necessarily a complete listing of components, elements or parts of the subject or subjects of the verb.

Various embodiments and aspects of the present invention as delineated hereinabove and as claimed in the claims section below find experimental support in the following examples.

### EXAMPLES

### Materials and Methods

### Microalgea growth and cultivation

*Phaeodactylum* microalgae were maintained in a defined artificial seawater medium which was developed from a growth medium (JONES, R. F., H. L. SPEER, AND W. KURY. 1963) used for the culture of the red alga *Porphyridium cruentum.* This modified salt-water medium, designated SW, contains per liter of H₂0: 27 gram (g) of NaCl, 6.6 g of MgSO₄-7H₂O, 5.6 g of MgCl₂^{∗} 6H₂O, 1.5 g of CaCl₂ - 2H₂O, 1.0 g of KNO₃, 0.07 g of KH₂PO4, 0.04 g of NaHCO₃, 1 ml of "iron stock solution" (18.6 g of Na2EDTA and 2.4 g of FeCl₃ 6H₂O/liter, pH 7), and 1 ml of "microelements" stock solution (40 mg of ZnCl₂, 600 mg of H3BO3, 15 mg of CoCl₂-6H₂O, 40 mg of CuCl₂-2H₂O, 488 mg of MnCl₂A4H₂O, and 37 mg of (NH4)6MoO₂₄-4H₂O per liter).

*Phaeodactylum* microalgae were cultivated at 20°C. Air, supplemented with 2% C02, was bubbled to maintain the culture pH at 7.5±0.5. The culture was harvested upon reaching a minimum biomass of 3.5 gram/Liter.

### Determination of fucoxanthin content in biomass and oleoresin

70-80 mg of biomass samples, or 20-25 mg of 5% fucoxanthin oleoresin samples, was diluted with 20 ml of methanol by sonication for 5 min in a 25 ml volumetric flask. After sonication and cooling to ambient temperature, the volume was adjusted to the final volume (25 ml) with methanol. The solutions were filtered through a 0,22 µm PVDF syringe filter before analysis by HPLC (injected in triplicate). In addition Fucoxanthin reference standard (fucoxanthin, Lot: CDX-00006296-010 obtained from Chromadex, USA with standard purity of 98.9% (HPLC) solution was prepared by diluting with methanol at a concentration of 50 ppm. This solution was well mixed and filtered through a 0,22 µm PVDF syringe filter before analysis by HPLC (injected in triplicate). Fucoxanthin, fucoxanthin isomers, and other carotenoids were identified in the analysis based on the retention time of the compounds in the chromatograms and the corresponding absorbance spectrum.

### Example 1

### Effect of nitrogen source on fucoxanthin accumulation in P. tricornutum microalgae

*P. tricornutum* microalgae were cultivated for 3 days, on day 3, nitrogen was added in the form of KNO₃ or urea (CH₄N₂O) alternatively. The content of fucoxanthin was determined by HPLC on three time points. Result show that when cells are grown in the presence of 0.5 g/liter urea the percent of fucoxanthin by dry weight of the biomass (also referred to as dry weight %/DW) is increased.

**Table 1. Effect of nitrate on Fucoxanthin production**

| **Fucoxanthin [%/DW]** | | | |
|---|---|---|---|
| | day 3 | day 6 | day 8 |
| No additional nitrogen | 1.24 | 0.96 | 0.77 |
| KNO3 | 1.25 | 1.3 | 1.26 |
| Urea | 1.29 | 1.54 | 1.6 |

### Example 2

### Effect of salt concentration on fatty acid accumulation in P. tricornutum microalgae

*P. tricornutum* microalgae were cultivated in the presence of different concentrations of sodium chloride (NaCl). Results show that when sodium chloride concentration in the medium was reduced from 27 g/liter to 9 g/liter the percentage of PA, AA, DPA, DHA and EPA from total fatty acid was increased (see table 2).

**Table 2. Effect of sodium chloride on total fatty acids (TFA) accumulation**

| **Fatty acid** | **Units** | **NaCl concentration** | |
|---|---|---|---|
| | | **9 gram/Liter** | **27 gram/Liter** |
| **Palmitoleic acid** | %/TFA | 19.4 | 17.2 |
| | %/DW | 2.5 | 2.6 |
| **AA** | %/TFA | 3.5 | 2.3 |
| | %/DW | 0.5 | 0.3 |
| **EPA** | %/TFA | 26 | 20.5 |
| | %/DW | 3.4 | 3.1 |
| **DPA** | %/TFA | 3.3 | 2.5 |
| | %/DW | 0.4 | 0.4 |
| **DHA** | %/TFA | 1.2 | 0.2 |
| | %/DW | 0.8 | 0.1 |
| **TFA** | %/DW | 13.1 | 15.2 |

### Example 3

### Biomass content of P. tricornutum microalgae

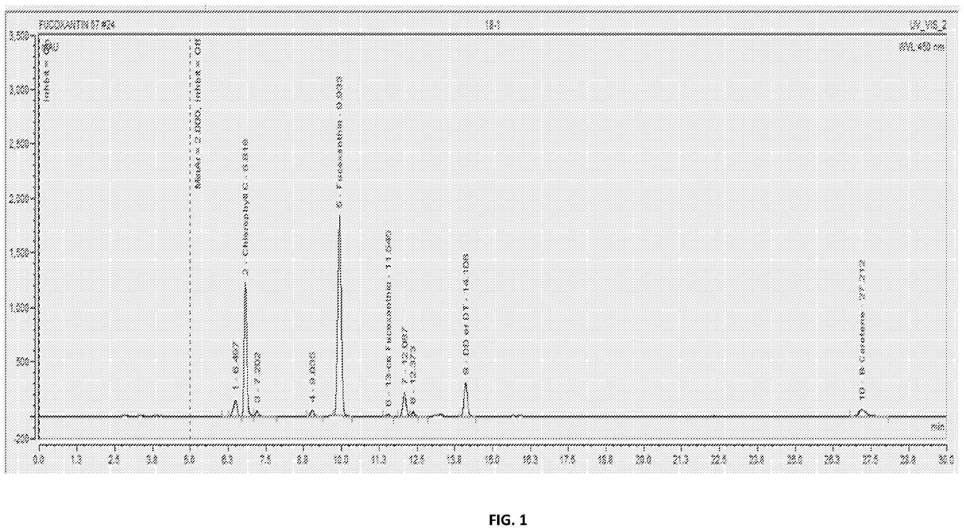

*P. tricornutum* microalgae were cultivated and harvested. The biomass content was examined by HPLC. A HPLC spectrum of the extract recorded at 450 nm is shown in FIG. 1.

The biomass content was analyzed and the calculated content in dry biomass is summarized in table 3.

**Table 3. P. tricornutum microalgae biomass content**

| | **units** | **Value** | **Units** | **Value** |
|---|---|---|---|---|
| Fuco | | | %/DW | 1.7-2 |
| Total Fatty Acids/DW | | | %/DW | 14.5 |
| C12:0 Lauric acid | %/total fat | 4-5 | %/DW | 0.03 |
| C14:0 Myristic acid | %/total fat | 12.2 | %/DW | 1.04 |
| C16:0 Palmitic acid | %/total fat | 0.2 | %/DW | 1.52 |
| C16:1 Palmitoleic acid+isomeres | %/total fat | 7.2 | %/DW | 3.10 |
| C16:3 Hexadecatrienoic acid (HTA) | %/total fat | 0.3 | %/DW | 2.25 |
| C18:0 Stearic acid | %/total fat | 17.6 | %/DW | 0.03 |
| C18:1-19 Oleic acid | %/total fat | 21.3 | %/DW | 0.06 |
| C18:2cis/trans | %/total fat | 0.6 | %/DW | 0.01 |
| C18:2 Linoleic acid | %/total fat | 7.3 | %/DW | 0.23 |
| C18:3 Alpha Linolenic acid | %/total fat | 1.1 | %/DW | 0.10 |
| C18:3 gamma-linolenic acid | %/total fat | 2.1 | %/DW | 0.04 |
| C18:4 Octadecatetraenic acid | %/total fat | 0.1 | %/DW | 0.09 |
| C20:0 Arachidic acid | %/total fat | 4.4 | %/DW | 0.06 |
| C20:1 Eicosenoic acid+isomers | %/total fat | 0.3 | %/DW | 0.03 |
| C20:2 Eicosodienoic acid+isomeres | %/total fat | 0.5 | %/DW | 0.04 |
| C20:4 Arachidonic Acid | %/total fat | 0.6 | %/DW | 0.36 |
| C20:5 Eicosapentaenic acid | %/total fat | 0.3 | %/DW | 4.04 |
| C22:0 Behenic acid | %/total fat | 0.1 | %/DW | 0.03 |
| C22:5 Docosapentaenic acid | %/total fat | 0.1 | %/DW | 0.19 |
| C22:6 Docosahexaenic acid | %/total fat | 0.2 | %/DW | 0.16 |
| C24:0 Lignoceric acid | %/total fat | 0.1 | %/DW | 0.62 |
| C24:1 Tetracosenoic acid+isomeres | %/total fat | 3.1 | %/DW | 0.14 |
| poly-unsaturated fatty acids | %/total fat | 25 | %/DW | 5.07 |
| Others | %/total fat | 0.5 | %/DW | < 0.1 |

### Example 4

### Extract of P. tricornutum microalgae

*P. tricornutum* microalgae were cultivated and harvested. The biomass was extracted by four alternative methods: ethanol extraction, SCF-CO2 extraction, SCF-CO2 and 2% ethanol extraction and SCF-CO2 followed by ethanol extraction (2 stages extraction). The contents of resulting extracts were compared to a control macro-algae (see table 4).

**Table 4. Comparison of extracts content**

| | **Units** | **Ethanol extract** | **"2 stage" extraction** | **SCF-CO2** | **SCF-CO2** | **SCF-CO2 +2% ethanol** | **Macro-algae extract** |
|---|---|---|---|---|---|---|---|
| **Fucoxanthin** | %/DW | 4.4 | 6.3 | 6.2 | 6.4 | 8.8 | 5.5 |
| **Purity** | % | 69.0 | 61.8 | 69.6 | 72.9 | 72.6 | 85.66 |
| **Total Fat** | gr/100gr | 52.4 | 81.0 | 72.5 | 70 | 79.5 | 91.4 |
| **C8:0 Caprilyc acid** | %/total fat | < 0.1 | < 0.1 | < 0.1 | < 0.1 | < 0.1 | 52.7 |
| **C10:0 Capric acid** | %/total fat | < 0.1 | < 0.1 | < 0.1 | < 0.1 | < 0.1 | 46.3 |
| **C14:0 Myristic acid** | %/total fat | 6 | 6.9 | 8.6 | 8.7 | 8.5 | < 0.1 |
| **C15:0 Pentadecanic acid** | %/total fat | 0.3 | 0.4 | 0.4 | 0.4 | 0.4 | < 0.1 |
| **C16:0 Palmitic acid** | %/total fat | 18.2 | 13.3 | 11.3 | 11.8 | 11.3 | 0.2 |
| **C16:1 Palmitoleic acid+isomers** | %/total fat | 22.7 | 27.9 | 24.6 | 25 | 24.3 | < 0.1 |
| **C18:0 Stearic acid** | %/total fat | 0.7 | 0.7 | 0.3 | 0.3 | 0.3 | < 0.1 |
| **C18:1 trans Elaidic acid** | %/total fat | 8.4 | 8.0 | 8.2 | 7.9 | 8.4 | < 0.1 |
| **C18:1-11 cis Vaccenic acid** | %/total fat | 0.9 | 0.8 | 0.5 | 0.6 | 0.6 | < 0.1 |
| **C18:1-19 Oleic acid** | %/total fat | 5.8 | 4.7 | 2 | 2.4 | 2.3 | 0.2 |
| **C18:2cis/trans** | %/total fat | 0.1 | 0.1 | < 0.1 | < 0.1 | 0.1 | < 0.1 |
| **C18:2 Linoleic acid** | %/total fat | 6.7 | 8.2 | 3.9 | 4.1 | 4.2 | < 0.1 |
| **C18:2 trans/trans** | %/total fat | 0.4 | 0.4 | 0.2 | 0.3 | 0.3 | < 0.1 |
| **C18:3 Alpha Linolenic acid** | %/total fat | 0.9 | 1.0 | 0.6 | 0.8 | 0.8 | 0.2 |
| **C18:3 gamma-linolenic acid** | %/total fat | 0.6 | 0.6 | 0.5 | 0.5 | 0.6 | < 0.1 |
| **C18:4 Octadecatetraeni c acid** | %/total fat | 0.4 | 0.3 | 0.4 | 0.4 | 0.4 | < 0.1 |
| **C20:4 Arachidonic Acid** | %/total fat | 3.1 | 4.0 | 3.9 | 3.8 | 3.9 | < 0.1 |
| **C20:5 Eicosapentaenic acid** | %/total fat | 22.4 | 20.4 | 32.8 | 31.5 | 32.3 | < 0.1 |
| **C22:0 Behenic acid** | %/total fat | 0.2 | 0.2 | < 0.1 | < 0.1 | < 0.1 | < 0.1 |
| **C22:6 Docosahexaenic acid** | %/total fat | 0.5 | 0.3 | 0.4 | 0.4 | 0.4 | < 0.1 |
| **C24:0 Lignoceric acid** | %/total fat | 1 | 0.8 | 0.3 | 0.3 | 0.3 | < 0.1 |
| **C24:1 Tetracosenoic acid+isomers** | %/total fat | 0.2 | 0.2 | 0.1 | 0.1 | 0.1 | < 0.1 |
| **Suturated Fatty Acids total** | %/total fat | 26.5 | 22.4 | 21.1 | 21.7 | 21 | 99.4 |
| **mono-unsaturated fatty acids** | %/total fat | 29.7 | 33.9 | 27.5 | 28.2 | 27.5 | 0.2 |
| **poly-unsaturated fatty acids** | %/total fat | 34.9 | 35.1 | 42.8 | 41.7 | 42.7 | 0.4 |
| **Total trans Fatty acids** | %/total fat | 8.9 | 8.7 | 8.6 | 8.4 | 8.8 | <0.1 |
| **Iodine** | ppm | < 0.2 | | | | | 0.9 |

### Example 5

### Fucoxanthin content of P. tricornutum microalgae Biomass and extract

The content of fucoxanthin was determined in five samples of Phaeodactylum tricornutum. Fucoxanthin, its isomers and other carotenoids were quantified by HPLC. The analyzed samples include: Biomass sample and 10% fucoxanthin oleoresin: NX2677.

Fucoxanthin, fucoxanthin isomers, and other carotenoids were identified in the analysis based on the retention time of the compounds in the chromatograms and the corresponding absorbance spectrum.

All of the biomass samples of *P. tricornutum* presented fucoxanthin concentrations above 1% weight/weight (% w/w), as summarized in table 5.

Fucoxanthin minor isomer presented in the chromatogram is tentatively identified as 13-cis or 13'-cis. This affirmation is done on basis of retention times and UV-vis absorption spectra. According to scientific literature cis isomers of carotenoids show an additional λ peak about 330 nm (Crupi et al., 2013). This peak represents about 5% of total fucoxanthin in the sample, as summarized in table 6.

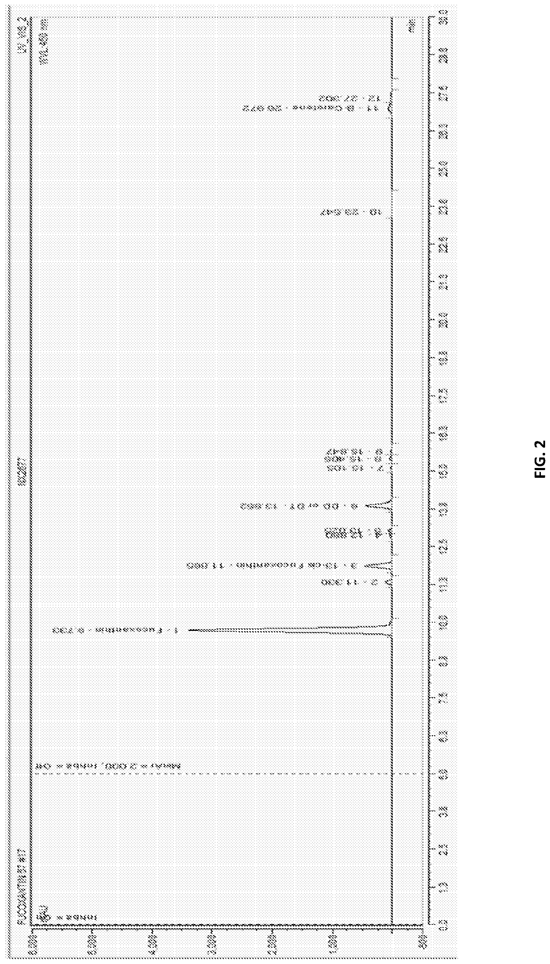

The presence of other carotenoids was also observed in the samples that have been identified as either diadinoxanthin or diatoxanthin and β-carotene (see Figure 1 and 2). This observation is sustained in the retention time, absorption spectra and scientific literature (Lavaud et al., 2002).

**Table 5. Fucoxanthin content in P. tricornutum samples**

| Sample | Compound | Result | unit |
|---|---|---|---|
| Biomass | *Fucoxanthin* | **1.31** ± 0.01 | %/DW |
| NX2677 Oleoresin | *Fucoxanthin* | **8.8** ± 0.5 | %/DW |

**Table 6. Relative levels of fucoxanthin isomers in P. tricornutum microalgae biomass**

| Compound | Relative % |
|---|---|
| all trans fucoxanthin | 95,26% |
| 13 cis or 13' cis fucoxanthin | 4,74% |
| total fucoxanthin | 100% |

### Example 6

### The content of P. tricornutum microalgae Biomass and extract

*P. tricornutum* microalgae were cultivated and harvested. Table 7a summarizes the dry biomass content of the *P. tricornutum* microalgae, the content of oleoresin obtained from the *P. tricornutum* microalgae, and the content of oleoresin obtained from macro-algae.

As demonstrated in table 7a, an oleoresin obtained from *P. tricornutum* contains 19.06 % eicosapentaenic acid (EPA), 2.38 % archidonic acid (AA), and 13.4 % palmitic acid (PA). Further, caprylic acid and capric acid constitute less than 0.02 and 0.05 of the content of the oleoresin obtained from *P. tricornutum.*

As further demonstrated in table 7a, the saturated fatty acids constitute 90.85% of the macro-algae extract and only 8.64% of the microalgae extract. fat content of an oleoresin obtained from macro-algae contains mostly caprilyc acid (48.17% from dry weight) and capric acid (42.32 % from dry weight), wherein unsaturated fatty acids constitute only 0.55% of the dry weight.

Notably, as demonstrated in table 7b the glucose content in oleoresin obtained from P. *tricornutum* was under the detection limit of the measuring device that was used (presented in the table as less than 0.1). Further the content of mono and disaccharides was also undetectable (presented in the table as less than 0.7).

**Table 7a. Biomass and oleoresin contents.**

| | *P. tricornutum* | | Macro-algae |
|---|---|---|---|
| | Dry biomass | Oleoresin | Oleoresin |
| Fucoxanthin [%] | 1.5-2 | 3.12 | 5.52 |
| Total fat [%] | 11.53 | 67.34 | 91.4 |
| Caprylic acid in product [%] | <0.02 | <0.02 | 48.17 |
| Capric acid in product [%] | 0.01 | 0.05 | 42.32 |
| PA in product [%] | 3.26 | 13.40 | <0.1 |
| AA in product [%] | 0.35 | 2.38 | <0.1 |
| EPA in product [%] | 3.53 | 19.06 | <0.1 |
| DHA in product [%] | 0.21 | 0.78 | <0.1 |
| Total UFA in product [%] | 9.47 | 58.71 | 0.55 |
| Total PUFA in product [%] | 4.77 | 28.39 | 0.37 |
| Total MUFA IN product [%] | 4.24 | 30.32 | 0.18 |
| Total saturated FA in product [%] | 3.34 | 8.64 | 90.85 |

**Table 7b. Biomass and oleoresin contents.**

| | *P. tricornutum* | |
|---|---|---|
| | Dry biomass | Oleoresin |
| Fucoxanthin [%] | 1.5-2 | 3.12 |
| Protein [%] | 40.90 | 4.84 |
| Total fat [%] | 11.53 | 67.34 |
| Total UFA in product [%] | 9.47 | 58.71 |
| Total PUFA in product [%] | 4.77 | 28.39 |
| Glucose [%] | 2.63 | <0.1 |
| Sum of mono and disaccharides | 2.63 | <0.7 |
| Sodium [%] | 1.73 | 0.25 |

### Example 7

### Effect of vitamin C and rosemary oil on Fucoxanthin stability

*P. tricornutum* microalgae were cultivated and harvested. Vitamin C was added to the resulting biomass to constitute 1% by weight of the biomass. Alternatively, rosemary oil was added to the resulting biomass to constitute 0.3% by weight of the biomass. The percentage of Fucoxanthin was determined prior to drying the biomass, in the dry biomass and 7 days post drying of the biomass. Table 8 presents a comparison of Fucoxanthin content of a biomass treated with vitamin C, rosemary oil or for an untreated biomass. Results demonstrate that Fucoxanthin is stabilized when either Vitamin C or rosemary oil are added to the biomass. Notably, in the presence of both vitamin C as well as rosemary oil reduction in Fucoxanthin in time (see last column).

**Table 8. Fucoxanthin stability under different condition**

| Treatment | % fucoxanthin of the biomass prior to drying of the biomass | % fucoxanthin of the dried biomass | % reduction of fucoxanthin level due to the drying process | % fucoxanthin of the dried biomass 7 days post the drying process | % reduction of fucoxanthin level |
|---|---|---|---|---|---|
| No addition | 1.71 | 1.69 | 1.0% | 1.36 | 20.6% |
| Rosemary oil | 1.70 | 1.73 | -1.5% | 1.40 | 17.2% |
| Vitamin C | 1.72 | 1.77 | -3.0% | 1.52 | 11.1% |

## Claims

1. A composition comprising: (i) a microalgae extract obtained from *Phaeodactylum tricornutum* microalgae, the microalgae extract comprising fucoxanthin and fatty acids, wherein monosaccharides and disaccharides constitute less than 0.7% by dry weight of said microalgae extract, and wherein said fucoxanthin constitutes more than 3% by dry weight of said microalgae extract; and (ii) vitamin E.

2. The composition of claim 1, wherein said fatty acids constitute more than 30% by dry weight of said microalgae extract.

3. The composition of claim 1, wherein glucose constitutes less than 0.1% by dry weight of said microalgae extract.

4. The composition of claim 1, being a nutritional supplement or a pharmaceutical composition.

5. The composition of claim 1, wherein said microalgae extract further comprises one or more carotenoids selected from diadinoxanthin, diatoxanthin and β-carotene, or isomers thereof.

6. The composition of claim 1, wherein said fatty acids are selected from the group consisting of: saturated fatty acids, mono-unsaturated fatty acids, poly-unsaturated fatty acids, trans fatty acids or any combinations thereof.

7. The composition of claim 6, wherein said saturated fatty acids are one or more fatty acids selected from the group consisting of: butyric acid, caproic acid, capric acid, lauric acid, myristic acid, pentadecenoic acids, heptadecenoic acid, stearic acid, behenic acid, lignoceric acid, or isomers thereof.

8. The composition of claim 6, wherein said mono-unsaturated fatty acids are one or more fatty acids selected from the group consisting of: myristoleic acid, palmitoleic acid, oleic acid, docosenic acid, or isomers thereof.

9. The composition of claim 6, wherein said poly-unsaturated fatty acids are one or more fatty acids selected from the group consisting of: eicosapentaenic acid (EPA), linoleic acid, alpha linolenic acid, gamma linolenic acid, docosapentaenic acid, docosahexaenic acid (DHA), arachidonic acid (AA), or isomers thereof.

10. The composition of claim 8 or 9, wherein any one of (i) said palmitoleic acid constitutes more than 18% by dry weight of said microalgae extract; (ii) said EPA constitutes more than 20% by dry weight of said microalgae extract; (iii) wherein said AA constitutes more than 0.3 % by dry weight of said microalgae extract; and (iv) said DHA constitutes more than 0.2% by dry weight of said microalgae extract.

11. The composition of any of claims 1-10, wherein iodine constitutes less than 0.2 ppm by dry weight of said microalgae extract.

12. The composition of any of claims 1-11, wherein heavy metals constitute less than 10 ppm by dry weight of said microalgae extract.

13. The composition of any one of claims 1-12, wherein said vitamin E constitutes between 0.1% to 5% by weight of the microalgae extract.

## Patentansprüche

1. Zusammensetzung, umfassend: (i) einen Mikroalgenextrakt, der aus *Phaeodactylum tricornutum* Mikroalgen erhalten wurde, wobei der Mikroalgenextrakt Fucoxanthin und Fettsäuren umfasst, wobei Monosaccharide und Disaccharide weniger als 0,7 % des Trockengewichts des Mikroalgenextrakts ausmachen und wobei das Fucoxanthin mehr als 3 % des Trockengewichts des Mikroalgenextrakts ausmacht; und (ii) Vitamin E.

2. Zusammensetzung nach Anspruch 1, wobei die Fettsäuren mehr als 30 % des Trockengewichts des Mikroalgenextrakts ausmachen.

3. Zusammensetzung nach Anspruch 1, wobei Glucose weniger als 0,1 % des Trockengewichts des Mikroalgenextrakts ausmacht.

4. Zusammensetzung nach Anspruch 1, die ein Nahrungsergänzungsmittel oder eine pharmazeutische Zusammensetzung ist.

5. Zusammensetzung nach Anspruch 1, wobei der Mikroalgenextrakt ferner ein oder mehrere Carotinoide, ausgewählt aus Diadinoxanthin, Diatoxanthin und β-Carotin, oder Isomeren davon, umfasst.

6. Zusammensetzung nach Anspruch 1, wobei die Fettsäuren ausgewählt sind aus der Gruppe bestehend aus: gesättigten Fettsäuren, einfach ungesättigten Fettsäuren, mehrfach ungesättigten Fettsäuren, trans-Fettsäuren oder jeden beliebigen Kombinationen davon.

7. Zusammensetzung nach Anspruch 6, wobei die gesättigten Fettsäuren eine oder mehrere Fettsäuren sind, ausgewählt aus der Gruppe bestehend aus Buttersäure, Capronsäure, Caprinsäure, Laurinsäure, Myristinsäure, Pentadecensäuren, Heptadecensäuren, Stearinsäure, Behensäure, Lignocerinsäure, oder Isomeren davon.

8. Zusammensetzung nach Anspruch 6, wobei die einfach ungesättigten Fettsäuren eine oder mehrere Fettsäuren sind, ausgewählt aus der Gruppe bestehend aus Myristoleinsäure, Palmitoleinsäure, Ölsäure, Docosensäure, oder Isomeren davon.

9. Zusammensetzung nach Anspruch 6, wobei die mehrfach ungesättigten Fettsäuren eine oder mehrere Fettsäuren sind, ausgewählt aus der Gruppe bestehend aus Eicosapentaensäure (EPA), Linolsäure, Alpha-Linolensäure, Gamma-Linolensäure, Docosapentaensäure, Docosahexaensäure (DHA), Arachidonsäure (AA), oder Isomeren davon.

10. Zusammensetzung nach Anspruch 8 oder 9, wobei (i) die Palmitoleinsäure mehr als 18 % des Trockengewichts des Mikroalgenextrakts ausmacht; (ii) die EPA mehr als 20 % des Trockengewichts des Mikroalgenextrakts ausmacht; (iii) wobei die AA mehr als 0,3 % des Trockengewichts des Mikroalgenextrakts ausmacht; und (iv) die DHA mehr als 0,2 % des Trockengewichts des Mikroalgenextrakts ausmacht.

11. Zusammensetzung nach einem beliebigen der Ansprüche 1 - 10, wobei Jod weniger als 0,2 ppm, bezogen auf das Trockengewicht des Mikroalgenextrakts, ausmacht.

12. Zusammensetzung nach einem beliebigen der Ansprüche 1 - 11, wobei Schwermetalle weniger als 10 ppm, bezogen auf das Trockengewicht des Mikroalgenextrakts, ausmachen.

13. Zusammensetzung nach einem beliebigen der Ansprüche 1 - 12, wobei das Vitamin E zwischen 0,1 und 5 Gew.-% des Mikroalgenextrakts ausmacht.

## Revendications

1. Composition comprenant : (i) un extrait de microalgues obtenu à partir de microalgues *Phaeodactylum tricornutum,* l'extrait de microalgues comprenant de la fucoxanthine et des acides gras, dans laquelle des monosaccharides et des disaccharides constituent moins de 0,7 % en poids sec dudit extrait de microalgues, et dans laquelle ladite fucoxanthine constitue plus de 3 % en poids sec dudit extrait de microalgues ; et (ii) de la vitamine E.

2. Composition selon la revendication 1, dans laquelle lesdits acides gras constituent plus de 30 % en poids sec dudit extrait de microalgues.

3. Composition selon la revendication 1, dans laquelle le glucose constitue moins de 0,1 % en poids sec dudit extrait de microalgue.

4. Composition selon la revendication 1, qui est un complément alimentaire ou une composition pharmaceutique.

5. Composition selon la revendication 1, dans laquelle ledit extrait de microalgues comprend en outre un ou plusieurs caroténoïdes choisis parmi la diadinoxanthine, la diatoxanthine et le β-carotène, ou des isomères de ceux-ci.

6. Composition selon la revendication 1, dans laquelle lesdits acides gras sont choisis dans le groupe comprenant : acides gras saturés, acides gras mono-insaturés, acides gras poly-insaturés, acides gras trans ou toute combinaison de ceux-ci.

7. Composition selon la revendication 6, dans laquelle lesdits acides gras saturés sont un ou plusieurs acides gras choisis dans le groupe comprenant : acide butyrique, acide caproïque, acide caprique, acide laurique, acide myristique, acides pentadécénoïques, acide heptadécénoïque, acide stéarique, acide béhénique, acide lignocérique, ou des isomères de ceux-ci.

8. Composition selon la revendication 6, dans laquelle lesdits acides gras mono-insaturés sont un ou plusieurs acides gras choisis dans le groupe comprenant : acide myristoléique, acide palmitoléique, acide oléique, acide docosénique, ou des isomères de ceux-ci.

9. Composition selon la revendication 6, dans laquelle lesdits acides gras polyinsaturés sont un ou plusieurs acides gras choisis dans le groupe comprenant : acide eicosapentaénique (EPA), acide linoléique, acide alpha linolénique, acide gamma linolénique, acide docosapentaénique, acide docosahexaénique (DHA), acide arachidonique (AA), ou des isomères de ceux-ci.

10. Composition selon la revendication 8 ou 9, comprenant un quelconque parmi (i) ledit acide palmitoléique constitue plus de 18 % en poids sec dudit extrait de microalgues ; (ii) ledit EPA constitue plus de 20 % en poids sec dudit extrait de microalgues ; (iii) ledit AA constitue plus de 0,3 % en poids sec dudit extrait de microalgues ; et (iv) ledit DHA constitue plus de 0,2 % en poids sec dudit extrait de microalgues.

11. Composition selon l'une quelconque des revendications 1 à 10, dans laquelle l'iode constitue moins de 0,2 ppm en poids sec dudit extrait de microalgues.

12. Composition selon l'une quelconque des revendications 1 à 11, dans laquelle les métaux lourds constituent moins de 10 ppm en poids sec dudit extrait de microalgues.

13. Composition selon l'une quelconque des revendications 1 à 12, dans laquelle ladite vitamine E constitue entre 0,1 % et 5 % en poids de l'extrait de microalgues.
